# EUROPEAN PATENT SPECIFICATION

(11) **EP 2 788 761 B1**
(45) Date of publication and mention of the grant of the patent: **28.02.2018**
(21) Application number: 12855270.0
(22) Date of filing: 28.09.2012
(51) Int. Cl.: A61M 1/34, G01N 33/53, C07K 16/18

(54) **REDUCTION OF GALECTIN-3 LEVELS BY PLASMAPHERESIS**
REDUKTION VON GALECTIN-3-KONZENTRATIONEN DURCH PLASMAPHERESE
RÉDUCTION DES TAUX DE GALECTINE-3 PAR PLASMAPHÉRÈSE

(30) Priority: 08.12.2011 US 201161568210 P
(43) Date of publication of application: 15.10.2014
(60) Divisional application: 16184096.2
(73) Proprietor: ELIAZ THERAPEUTICS, INC., Sebastopol CA 95473 (US)
(72) Inventor: ELIAZ, Isaac, Santa Rosa, CA 95401 (US)
(74) Representative: J A Kemp
(86) International application number: PCT/US2012/057749
(87) International publication number: WO 2013/085604

(56) References cited:
- WO-A1-2010/006576
- WO-A2-2010/065765
- US-A1- 2008 089 959
- US-A1- 2011 104 722
- US-A1- 2011 294 755
- US-B2- 7 846 650

## Description

### BACKGROUND OF THE INVENTION

### Field of the Invention

This invention pertains to treatment of disease and biological conditions mediated at least in part by one or more galectins. Galectins are a family of lectins (sugar binding proteins) that are characterized by having at least one carbohydrate recognition domain (CRD) with an affinity for beta-galactosides. These proteins were recognized as a family only recently, but are found throughout the animal kingdom, and are found in mammals, birds, amphibians, fish, sponges, nematodes and even fungi. This application focuses on galectins in mammals, and in particular, humans. Although the invention herein may be employed with both companion animals (e.g., pets such as dogs and cats) and commercial animals (such as cows, pigs and sheep) the methods and subject matter addressed herein are particularly focused on the treatment of humans.

Galectins mediate and modulate a wide variety of intracellular and extracellular functions, and thus are both expressed within the cell and frequently targeted to a specific cytosolic site, and secreted from the cell, for distribution extra-cellularly, as a component of human plasma. Among the many functions that are mediated by extracellular galectins are inflammation, fibrosis formation, cell adhesion, cell proliferation and metastatic formation (cancer) and immunosuppression.

Galectins are a family of fifteen (15) carbohydrate-binding proteins (lectins) highly conserved throughout animal species. Most galectins are widely distributed, though galectin -5, -10 and -12 show tissue-specific distribution. While galectins are variably expressed by all immune cells, they are upregulated in activated B and T cells, inflammatory macrophages, natural killer (NK) cells, and FoxP3 regulatory T cells. Galectins contain a variety of structural arrangements, but a relatively conserved carbohydrate recognition domain (CRD). The majority of galectins display a single CRD, and are biologically active as monomers (galectin-5,-7 and -10), or require homodimerization for functional activity (galectin-1,-2,-11,-13,-14 and-15). Alternatively, tandem-repeat-type galectins (galectin-4,-8,-9, and -12) contain two CRDs separated by a short linker peptide, while galectin-3 (chimeric type) has a single CRD fused to a non-lectin domain that can be complexed with other galectin-3 monomers to form an oligomeric pentamer. Of note, some galectins, such as galectin-10, bind to mannose-containing glycans. Among the family of galectins -1, -3, and -9 are particularly important as potential therapeutic targets, and -2,-4,-5,-6,-7,-8,-10, -11,-12,-13,-14, and -15 also appear implicated in a variety of biological pathways associated with morbidity and mortality.

Thus, galectin-7 has been implicated in the development of certain forms of cancer. St. Pierre et al, Front. Biosci., 1:17, 438-50 (2012) and in a variety of specific cancers, including gal-2, -4 and -8 in the context of colon and breast cancer, Barrow et al, Clin. Cancer Res,. 15;17 (22) 7035-46 (2011). Squamous cell carcinoma of the tongue, Alves et al., Pathol. Res. Tract. 15;207 (4) 236-40 (2011) has been shown to be associated with elevated levels of gal-1, -3 and -7, while cervical squamous carcinoma has been shown linked to gal-7 levels, Zhu et al, Int. J. Cancer, (Aug., 2012). A number of galectins, including gal-15, gal-13 and gal-10 have been demonstrated to be linked to implantation and pregnancy concerns. See, e.g., Than et al, Eur. J. Biochem. 271(6) 1065-78 (2004), Lewis et al, Biol. Reprod. 77(6); 1027-36 (2007). A number of galectins, including gal-2, 3, 8 and others have been identified as correlating with various autoimmune disorders, such as lupus. Salwati et al, J. Infect. Dis. 1;202(1) 117-24 (2010), Pal et al, Biochim. Biophys. Acta., 1820 (10) 1512-18 (2012) and Janko et al, Lupus 21(7) :781-3 (2012). Elevated levels of a number of galectins, including gal-3, are associated with inflammation and fibroses encountered in wound healing and the like. Gal et al, Acta. Histochem. Cytochem. 26:44(5); 191-9 (2011).

Quite obviously, mediation of inflammatory and fibrotic pathways makes galectins critical elements of a wide variety of disease, injury and trauma related phenomena. In many cases, the presence of unwanted concentrations of galectins can aggravate a disease condition or trauma situation, or interfere with attempts to treat diseases, such as cancer or congestive heart failure. Among the family of galectins recognized as active intracellularly in humans, galectin-1, galectin-3 and galectin-9 are of particular interest. As indicated above, these proteins are generally referred to, and referred to herein as, gal-1, gal-3 and gal-9. A wide variety of conditions in humans, ranging from problems in conceiving to asthma to chronic heart failure to cancer to viral infection to stroke and beyond are mediated or aggravated by higher than normal concentrations of galectins. Thus, among other galectins, gal-3 is particularly prominent in fibrosis, inflammation and cell proliferation, while gal-1 also plays a role in the immunosuppression required for a successful pregnancy. Gal-1 is also thought to be involved in the differentiation of nerve cells. Gal-9 has been shown to be involved in the control of lesions arising from immunoinflammatory diseases, and is generally implicated in inflammation - gal-9 apparently plays a role in eosinophil recruitment in inflammatory sites. It also appears to mediate apoptosis in certain activated cells.

While the discussion herein is applicable to circulating active gal-1, gal-3 and gal-9, and galectins in general where elevated circulating galectin levels are associated with disease or injury conditions, more has been elucidated about the role of gal-3 in disease and trauma progression than any of the other galectins, and so it is exemplified herein. More specifically, this invention focuses on the removal of gal-3 from mammalian, particularly human, plasma. Gal-3 has been shown to be involved in a large number of biological processes, many of which are related to disease states of various kinds. Binding and blocking activity of gal-3 in the circulation, or removal of large amounts of gal-3 from circulation may therefore improve existing medical treatments, suppress and/or reduce inflammation and fibrosis resulting from others, and make it possible to intervene in various disease states not otherwise easily treated. The invention is equally applicable to the reduction in circulating levels of other galectins to address conditions mediated by those galectins.

This invention makes use of plasmapheresis, sometimes referred to as therapeutic plasma exchange, to control levels of gal-3, and more specifically biologically active galectin, in circulation. Plasma is lead through a fluid pathway and either intermixed with a gal-3 binding agent which can be separated from the plasma, or returned to the body with blocked inactivated gal-3, or lead past a solid support which binds gal-3, the plasma being subsequently returned to the body with a reduced level of gal-3.

### Related Art

In U.S. Patent Application Serial No. 13/153,648 (U.S. Patent Publication US-2011-0294755 A1) a method of treating cell proliferation conditions, inflammation and aggravated fibroses is disclosed which involves the administration of an agent that can bind circulating gal-3, such as modified citrus pectin, or MCP, a citrus pectin which has a reduced molecular weight of twenty thousand (20,000) Daltons or less, preferably ten thousand (10,000) Daltons or so. MCP is available commercially from EcoNugenics of Santa Rosa, California and is discussed in U.S. Patent Nos. 6,274,566 and 6,462,029.
WO 2010/065765 describes methods, devices and systems for capturing biomarkers using affinity capturing devices.

### Background of the Technology

Gal-3 is approximately 30 kDa and, like all galectins, contains a carbohydrate-recognition-binding domain (CRD) of about one hundred thirty (130) amino acids that enable the specific binding of β-galactosides. Gal-3 is encoded by a single gene, LGALS3, located on chromosome 14, locus q21-q22. This protein has been shown to be involved in a large number of biological processes. The list set forth herein is exemplary only as new situations and roles for gal-3 are continually being revealed. Among the biological processes at the cellular level that have been shown to be mediated, at least in part, by gal-3, are cell adhesion, cell migration, cell invasion, cell activation and chemoattraction, cell growth and differentiation, cell cycle, and apoptosis.

Given gal-3's broad biological functionality, it has been demonstrated to be involved in a large number of disease states or medical implications. Studies have also shown that the expression of gal-3 is implicated in a variety of processes associated with heart failure, including myofibroblast proliferation, fibrogenesis, tissue repair, inflammation, and ventricular and tissue remodeling. Elevated levels of gal-3 in the blood have been found to be significantly associated with increased morbidity and mortality. They have also been found to be significantly associated with higher risk of death in both acute decompensated heart failure and chronic heart failure populations.

Various investigations have shown elevated levels of gal-3 to aggravate a wide variety of disease conditions associated with cell proliferation. High levels of gal-3 are linked to cancer growth and cancer progression to a metastatic stage in a stunning variety of cancers. A number of cancers have been specifically linked to or associated with elevated gal-3 levels, including liver cancer, kidney cancer, breast cancer, prostate cancer, colon cancer, thyroid cancer, cancer of the gallbladder, nasopharyngeal cancer, lymphocytic leukemia, lung cancer, melanoma, multiple myeloma, glioblastoma multiforme, uterine cancer, ovarian cancer, cervical cancer, brain cancer and others. Elevated gal-3 levels have also been shown to interfere with or suppress conventional antineoplastic regimens, such as chemotherapeutic treatments like cis-platinum, doxorubicin and related chemotherapeutics.

Inflammation is a commonly encountered body condition - a natural response of the body to a variety of diseases and trauma. As with the other conditions noted above, gal-3 levels above normal levels are implicated in a wide variety of situations where harmful inflammation is encountered. Again, the list of conditions and disease states is too extensive to exhaust every possibility, but inflammatory conditions associated with elevated gal-3 levels include aggravated inflammation associated with non-degradable pathogens, autoimmune reactions, allergies, ionizing radiation exposure, diabetes, heart disease and dysfunction, atherosclerosis, bronchial inflammation, intestinal ulcers, intestinal inflammation of the bowels, cirrhosis-associated hepatic inflammation, parasitic infection associated inflammation, inflammation associated with viral infection, inflammation associated with fungal infection, inflammation associated with arthritis, with multiple sclerosis and psoriasis. Again, while inflammation is a pathway frequently employed by the body in responding to any number of challenges, elevated levels of gal-3 have been found to aggravate the inflammation, causing damage and injury leading to morbidity or mortality in a wide variety of situations that are otherwise manageable, including inflammation due to heavy metal poisoning and similar toxins, stroke and related ischemic injuries, liver inflammation due to acetaminophen, a number of T-cell mediated responses generally involved in autoimmune diseases and the like. Gal-3 is also involved with kidney injury and kidney disease, hepatitis, pulmonary hypertension and fibrosis, diabetes, and gastrointestinal inflammatory conditions such as Ulcerative colitis, Chrone's, Celiac, and others.

As noted, elevated levels of circulating, active gal-3 are associated with, and apparently aggravate, a number of inflammatory conditions, including those contributing to heart, kidney, lung, and liver disease. Gal-3 is also associated with a fibrotic formation, particularly in response to organ damage. Higher levels of circulating gal-3 are found to induce pathogenic fibroses in cardiovascular disease, gastroenterological disease, cardiovascular trauma, renal tissue trauma, brain trauma, lung trauma, hepatic tissue trauma, tissue damage due to radiation therapy and diseases and conditions of connective tissue and skin such as systemic sclerosis.

Accordingly, the art is replete with observations that elevated levels of gal-3, as well as gal-1 and gal-9, can complicate or exacerbate a wide variety of disease and injury conditions. It would be of value to find a way to control inflammation and formation of fibroses, where the inflammation and fibroses are injurious, particularly in the environments described above, and notably in cardiac care and other organ tissue disease and trauma. By the same token, it would be of value to control the cellular responses mediated by gal-3 that accelerate cell proliferation and transformation, including the formation and growth of tumors, the transformation of cancer cells and metastatic spread of cancer. Another goal in the art is to avoid the problem posed by the interference in the treatment of cancer by conventional agents, like bleomycin, Adriamycin, doxorubicin, cyclophosphamide and cyclosporine. Some of the side effects caused by these agents are gal-3 mediated, and can be addressed and ameliorated by the invention. Elevated gal-3 levels also appear to interfere with pharmaceuticals used in other applications, such as the antiarrhythmic drug amiodarone, and statin drugs.

Plasmapheresis is a blood separation technology, where blood is diverted from the body through a needle or catheter to a separator which removes blood cells and returns them to the body, leaving a plasma. This type of technique has been used historically in the treatment of autoimmune diseases, where the antibodies at issue are removed by contacting the plasma with the ligands to which they bind. The plasma is then augmented as required, with anticoagulants, therapeutics and associated elements, and returned to the body.

An early form of apparatus for plasmapheresis is set forth in U.S. Patent No. 3,625,212, which describes measures to ensure return of treated plasma, as well as the separated blood cells, to the proper donor. U.S. Patent No. 4,531,932 addresses plasmapheresis by centrifugation, the method used to separate out the red blood cells, on a rapid and near-continuous basis. U.S. Patent Nos. 6,245,038 and 6,627,151 each describe a variety of methods of separating out plasma contents and returning the treated plasma to the patient after first removing red blood cells, in general, to reduce blood viscosity by removal of high molecular weight protein. While the invention that is the subject of this application focuses on the reduction in galectin-3 circulating levels and not high molecular weight proteins or directly addressing viscosity, available plasmapheresis techniques and apparatus, as disclosed in these four (4) patents, may generally be employed in this invention.

Prior to the development of this invention, those of skill in the art had experimented with the reduction of gal-3 levels in various respects. Thus, the activity of gal-3 in aggravating or promoting cancer, as well as the ability of a cancer to metastasize, is widely commented on in the literature following 2006. These literature findings stress repeatedly the importance of binding or reducing the circulating concentration or titer of gal-3, and/or inactivating gal-3 through gal-3 binders such as PectaSol-C MCP. See, for example, Wang et al, Cell Death and Disease, 1-10 (2010) (gal-3 inhibition promotes treatment) and Yu et al, J. Biol. Chemistry, Vol. 282, 1, pp. 773-781 (2007) establishing that gal-3 interactions may enhance formation of cancer or transformation of metastatic cancer.

As disclosed and claimed in U.S. Patent No. 6,274,566, Gal-3 binders such as Modified Citrus Pectin and other compounds can bind to circulating tumor cells (CTC's) and prevent them from creating new metastasis. These CTC's are often implicated in mutations and a more aggressive disease. Cancer stem cells that may also be circulating and get stimulated under conditions of stress and inflammation, provide gal-3 another mechanism for aggravating cancer. The method of these prior cases may be used in conjunction with the invention of this application. In particular, when there are a high number of gal-3 molecules circulating in the blood stream it makes it more difficult for the gal-3 binders to target these CTCs. In this respect, gal-3 molecules serve as decoy molecules. The decoy prevents, in this particular application of the invention, binding of the cancer cells in the circulatory or lymph system, as opposed to tissue level gal-3.

As a consequence, reports link acceleration of cancer formation and transformation to circulating gal-3 concentrations, and suggest that reducing gal-3 circulating concentrations, reducing its free expression or otherwise reducing available gal-3 or gal-3 interactions improves cancer prognosis. Zhao et al, Cancer Res, 69, 6799-6806 (2009), Zhao et al, Molecular Cancer 9, 154, 1-12 (2010) and Wang et al, Am. J. of Pathology, 174, 4, 1515-1523 (2009) wherein siRNA-induced reduction of gal-3 is shown to slow the course of prostate cancer. Similarly, high-risk bladder cancer recurrence and prognosis is related indirectly to gal-3 levels. Rodriguez et al, J. Curr. Opin. Urol. 22(5):415-20 (2012) and Raspollini et al, Appl. Immunohistochem. Mol. Morphol. (July 2012). Clearly, there is substantial literature that supports the conclusion that reducing circulating gal-3, either by blocking its expression, or by binding it, is important in controlling cancer, both in tissue and in circulation.

Circulating gal-3 is empirically implicated in a wide variety of biological conditions, however. Cardiac fibrosis is gaining significant attention as a complicating risk factor in cardiac disease, and in particular, chronic heart failure (CHF). Lok et al, Clin. Res. Cardiol, 99, 323-328 (2010). DeFillipi et al, U.S. Cardiology, 7,1, 3-6 (2010) clearly indicate that circulating gal-3 is an important factor in fibrosis of many organs and organ systems, and that reducing circulating gal-3 may have an important role in remediating cardiac injury and progression to heart failure (HF). Similarly, Psarras et al, Eur. Heart J., April 26, 2011 demonstrate that reduction in gal-3 levels in the myocardium may reduce fibrosis in the heart and improve outlook. De Boer et al, Ann. Med., 43,1, 60-68 (2011) identify gal-3 as a key indicator in cardiac health. Shash et al, Eur J. Heart Fail., 12, 8, 826-32 (2011) identify gal-3 levels as a key agent in heart failure through fibrosis. De Boer et al., Eur. J. Heart Fail., 11, 9, 811-817 (2009) link an increase in gal-3 expression and presence to heightened fibrosis, and heart failure. The same article links gal-3 to inflammation. Inflammation is the hallmark of arteriosclerosis and therefore gal-3 levels also contribute to coronary artery disease, peripheral artery disease, strokes, and vascular dementia.

Fibrosis and inflammation, both mediated to some degree by gal-3 (cellular or circulating) are implicated in a variety of conditions of the mammalian body, not just cardiac injury and heart failure. The binding of gal-3 achieved by administration of low molecular weight pectins (10,000-20,000 Daltons molecular weight such as PectaSol-C MCP) is effective in reducing trauma due to kidney injury. Kolatsi-Jannou et al, PlusOne, 6, 4, e18683 (2011). Reducing circulating gal-3 levels may be effective in reducing fibrosis in the lungs and associated asthma. Cederfur et al, Biochim. Biophys. Acta. 1820(9):1429-36 (2012). The reduction in circulating gal-3 levels is also indicated to reduce inflammation associated with type 2 diabetics, and similar metabolic diseases, as well as obesity. Weigert et al, J. Endocrinol. Metab. 95, 3,1404-1411(2010). Thus, high levels of gal-3 have been linked to thyroid cancer, Sethi et al, J. Exp. Ther. Oncol., 8, 4,341-52 (2010) and reduction of gal-3 expression and circulation may delay or reduce tumor cell transformation. Chiu et al, Am J. Pathol. 176, 5, 2067-81 (2010).

As noted, gal-3 is implicated in a wide variety of biological conditions, and a reduction in gal-3 activity, such as that which can be achieved by gal-3 binding with PectaSol-C MCP and similar low molecular weight pectins may be of value in treating gastric ulcerative conditions. Srikanta, Biochimie, 92, 2, 194-203 (2010). Kim et al, Gastroenterology, 138, 1035-45 (2010) indicate that reducing gal-3 levels may be of therapeutic value in reducing gastric cancer progression. By the same methodology, reducing gal-3 levels sensitizes gastric cancer cells to conventional chemotherapeutic agents. Cheong et al, Cancer Sci., 101, 1, 94-102 (2010). Gal-3 is implicated in a wide variety of gastrointestinal conditions. Reducing gal-3, by binding for example, may reduce inflammation in the gut mucosa, making MCP an important agent for treatment of ulcerative colitis, non-specific colitis and ileitis, Crohn's disease, Celiac disease, and gluten sensitivity. Fowler et al, Cell Microbiol., 81,1,44-54 (2006).

Biliary artesia, a liver disease, is associated with extensive fibrosis of the liver linked with elevated gal-3 levels. Honsawek et al, Eur. J. Pediatr. Sung., April, 2011. Reduction of gal-3 levels resulted in a general improvement in hepatic health, including reducing inflammation, hepatocyte injury and fibrosis. Federici et al, J. Heptal., 54, 5, 975-83 (2011). See also, Liu et al, World J. Gastroenterol. 14,48, 7386-91 (2008) which reported, following Applicant's teaching in 2005 and 2006 to administer low molecular weight MCP, that MCP inhibited liver metastases of colon cancer and reduced gal-3 concentrations. MCP, or other gal-3 binders, may be used for prevention of liver inflammation, liver fibrosis and liver cirrhosis as well as post-disease liver damage, including the various viral hepatitis diseases (A, B, C, and others) and may be used as well in the treatment of parasitic and chemical hepatitis, chemical liver damage, and others. Gal-3 levels are implicated in a wide variety of liver associated ailments. Thus, gal-3 may be important in the control of Niemann-Pick disease type C, which is a lysosomal disorder characterized by liver disease and progressive neurodegeneration. Cluzeau et al, Hum. Mol. Geent. 14; 21 (16) 3632-46 (2012). There is increasing evidence that elevated gal-3 levels are tied to acetaminophen-induced hepatotoxicity and inflammation. Radosavljeci et al, Toxicol. Sci., 127:609-19 (2012). Reduction in gal-3 levels may improve treatments. Dragomir et al, Toxicol. Sci. 127(2):609-19 (2012).

While administration of modified citrus pectin, or a similar binding agent, continues to be a promising therapy of inhibition of damage, and repair of damage, induced by gal-3, the inventor has continued to work to find other methods of providing faster or more profound relief. It has now been found that by selective use of certain gal-3 binding molecules, gal-3 and specifically biologically active gal-3 can be specifically removed from the plasma in significant amounts. Return of the plasma with a reduced titer of active gal-3 offers immediate opportunities for therapy and intervention that may be different from, or more profound than, the reduction achieved by administration of binding molecules to a mammal in need of same. By removing the circulating gal-3 molecules the invention removes these protective but potentially harmful molecules from the circulation. In addition, it allows targeted gal-3 blockers such as MCP, and possibly other oligo-saccharides and various pharmaceutical agents to be developed to better attach to the gal-3 on the cell surface and on the tissue level. As the expression of gal-3 is increased in the injured and inflamed tissue, such as remodeled cardiac muscle or cancer tissue, by removing the circulating gal-3, the gal-3 binding agent can more effectively bind to the Gal-3 in the target tissue.

### SUMMARY OF THE INVENTION

The present invention provides a galectin-3 (gal-3) binding molecule for use in a method of treating (a) cancer; or (b) fibroses; or (c) harmful inflammation in a mammal wherein the mammal is respectively (a) in need of inhibition of the growth or spread of cancer mediated at least in part by gal-3; or (b) in need of inhibition of development or extension of fibroses mediated by gal-3; or (c) in need of inhibition of inflammation mediated by gal-3; said method comprising:
conducting plasmapheresis on the blood of a mammal in need of reduction of circulating levels of gal-3 to reduce circulating levels of active gal-3, wherein said plasmapheresis is conducted so as to selectively remove gal-3 by contact with said gal-3 binding molecule, such that at least ten percent of the circulating gal-3 in the serum of that individual is removed by said plasmapheresis; wherein the gal-3 binding molecule comprises a gal-3 binding antibody or modified citrus pectin (MCP).

The invention relates to a gal-3 molecule for use in a method of removal of biologically active gal-3 from a mammal's circulation by plasmapheresis. The mammal may be a human, a primate, a model such as a rat or mouse, a commercial animal such as a cow or pig or goat, or a companion animal such as a dog or cat. Non-human mammalian animals for treatment include primates, both as models and as test beds for treatments and intervention that may benefit from removal of gal-3 from circulation. Removal is achieved by plasmapheresis, a process traditionally developed and used to remove antibodies from the circulation of those suffering from autoimmune disorders and the like.

In plasmapheresis, blood is removed from the patient, and blood cells are separated out from the plasma. The blood cells are returned to the body's circulation, diluted with fresh plasma or a substitute. Conventional plasmapheresis methods and medications that can include blood thinners are utilized. While in typical plasmapheresis, the plasma is run over proteins to which the target antibodies bind, in this particular case, the plasma is returned to the blood with the antibodies, cytokines, lymphocytes and other blood components, after having had gal-3 selectively removed or inactivated by contact with gal-3 binding molecules. In the case of autoimmune diseases, the removal of galectin-3 from the plasma can be an adjuvant therapy added to the traditional plasmapheresis performed for such patients.

This treatment may be used for all the conditions where gal-3 levels are elevated in the blood or serum or where expression of gal-3 in the tissues is too high. Tissues will shed excess gal-3 into the blood stream where it can be removed through this invention. Treatment can be varied depending on the patient, the severity of the condition and the rate of the mammalian patient's expression of gal-3. Ordinarily, treatment every two to four weeks is contemplated until the condition is resolved, but treatment may be daily where required, or at any frequency there between. Daily treatment includes one or more plasma exchange sessions in a given day, or continuous plasmapheresis over a multiple hour period in acute conditions. Treatment can be administered on an acute or a chronic basis. Advantageously, this treatment is combined with the administration of gal-3 blockers and inhibitors, such as disclosed in US-2011-0294755 A1. Although modified citrus pectin is a target inhibitor, other gal-3 inhibitors, such as other modified carbohydrates, including lactulosyl-1-leucine, Dermotte et al, Can. Res., 70 (19):476-88 (October 2010) as well as antibodies specific for gal-3, and other antagonists like very low molecular weight pectin weighing as low as 1KD, GCS-100, Streetly et al, Blood, 115(19):3939-48 (published February 26, 2010 as an abstract) may be used. GCS is a polysaccharide derived from MCP, as opposed to reduced MCP. A large variety of gal-3 binding antibodies are commercially available, from suppliers including abcam (ab2473), Novus Biologics (NB 100-91778) and Abgent (AJ13129). Other galectins-3 specific antibodies may be used. By removing large levels of plasma active gal-3 from the blood, the disease and injury due to inflammation or fibroses may be reduced, and the progression of cancer may be impeded. Similarly, conventional therapeutic treatments may be rendered more effective.

In a preferred embodiment, at the same time active gal-3 is removed, soluble TNF receptors, both R-1 and/or R-2 at different ratios based on the condition, are removed, through the same process, by running the plasma fluid over a bed of binding agents of TNF receptors. TNF can then directly target cancer cells or other targets as an effective treatment. The reduction of active gal-3 in both the circulation and the tissue level will allow TNF to exert its beneficial effects with a reduced amount of inflammation and fibrosis which limits its use. Wu et al, Arch. Dermatol. 20:1-7 (2012). The effective removal of serum gal-3 also enhances chemotherapy, particularly, but not exclusively, when combined with TNF receptor removal. Chemotherapy enhancement will take place by effective removal of serum gal-3, reducing drug resistance, even if no TNF receptors were removed from the circulation. Yamamato-Sugitani et al, PNAS, 18:108(42), 178468-73 (2012). Gal-3 interferes with platinum-based chemotherapy and other anti-cancer agents, and increases cell adhesion, and angiogenesis. Wu et al, Cell Oncol. 35(3):175-80 (2012). In addition, removal of gal-3, by plasmapheresis alone, or together with administration of circulating gal-3 binders like low molecular weight modified citrus pectin, may effectively treat the diseases and conditions addressed above. In addition, this can be further enhanced by combining it with other therapies, one example being chemotherapy in cancer.

Typical circulating gal-3 level averages for a Caucasian adult range from 7 on up to about 20 ng/ml, with a value of 12-15 nanograms of gal-3 per milliliter of serum being a representative and reported value. Patients at risk, including those with advanced illnesses, exhibit levels, without treatment, that can be much higher than that patient's average or normal level. In accordance with the present disclosure, individuals facing serious illness or continued disability due to gal-3 mediated fibrosis, gal-3 mediated inflammation, and cancer growth, transformation and metastases associated with elevated gal-3 levels are treated by plasmapheresis to achieve a significant reduction in circulating gal-3 titer.

By significant reduction in circulating gal-3 levels, inflammation and/or fibrosis due to trauma or disease condition can be controlled. Similar reductions in gal-3 levels can aid in the control of the growth, spread and the transformation of various kinds of cancer. In general, a reduction of circulating gal-3 of at least ten percent (10%) is necessary to achieve significant progress in gal-3 mediated fibroses, and even more may be required in acute conditions involving inflammation, fibroses due to trauma or aggressive cancer. In functional terms, the reduction of gal-3 should be sufficient to reduce or inhibit the impact of gal-3 levels on inflammation and fibroses, or cancer growth and transformation, in said patient. Reduction in circulating gal-3 of at least twenty percent (20%), and in some cases at least forty percent (40%) or even fifty percent (50%), may be required on a sustained basis. Severe situations may require reduction in circulating gal-3 levels in a mammalian patient of greater than fifty percent (50%) of that patient's circulating gal-3 titer, on up to seventy-five percent (75%) or even more. While some level of gal-3 in circulation is required for homeostasis, in acute situations, reductions at least by eighty percent (80%) of circulating gal-3, on up to near total removal of gal-3 from serum, may be called for, as that level is quickly replenished by the body. Acute situations can be found in all sorts of individuals, but a representative example is hepatic inflammation or transformation in an aggressive cancer like pancreatic cancer or small cell lung carcinoma.

The gal-3 levels in races other than Caucasians and subjects may vary, but the target is to reduce gal-3 levels below the appropriate normal value. Target levels can vary based on the condition, age, gender, and other therapies involved. As a general matter, treatment of the patient may begin with plasmapheresis designed to reduce the patient's gal-3 to a preselected value consistent with good health and homeostasis in that individual. In some cases, it may be necessary to repeat or extend that treatment to achieve even greater reductions.

This invention is straightforward in its application. It is recognizing how many different indications are served by this technology that is complex and startling. Blood is removed from the patient according to well established protocols generally used for plasmapheresis. See, generally, Samuels et al, editors, Office Practice of Neurology, 1996. The removed blood is treated to remove blood cells from the plasma. These blood cells, together with an additional volume of plasma or plasma substitute, are returned directly to the patient. In a single session, two to four liters of plasma may be removed, filtered, and replaced. The blood can also be recycled and recirculated extra corporally, and filtered as needed, for a number of times (continuously) until the desired reduction in serum levels of galectin-3 is achieved. Different serum levels can be targeted for different conditions. The blood cell-depleted plasma is then introduced to a chamber where gal-3 is removed or inactivated by binding antagonist, possibly creating a permanent bond that inactivates the gal-3. One of two alternative measures may be used to remove gal-3, although they may be combined. In a first alternative, the plasma is admixed with a particle which binds gal-3. Preferably, this is an antibody or similar ligand, or a polysaccharide derivative that is most preferably modified citrus pectin (MCP), but any agent that can bind gal-3 can be used. Methods of preparing low molecular weight pectins are known in the art, and set forth in U.S. -2011-0294755 A1.

The binding agent is modified to be complexed with an agent that is easily removed. In one embodiment, this is a magnetic particle. After providing for adequate circulation time, a magnetic field is applied to the fluid comprising the plasma and the MCP complex, and the bound gal-3 can be drawn off. Different filters that incorporate gal-3 binders can be used in the plasmapheresis process.

In certain conditions such as cancer, the circulating gal-3 can be viewed as a sort of decoy released by the cancer cells. It has a protective quality as it doesn't allow the host, and doesn't allow gal-3 binders such as MCP, to reach the target tissue where galecin-3 is over expressed. It also induces inflammation and fibrosis and makes it more difficult for the host to bind to the gal-3 in the tissue and cell surface level. Removing the circulating gal-3 provides both a therapeutic treatment on its own and allows other agents to bind and inactivate the gal-3 in the target tissue level. This is similar to TNF Alpha and circulating TNF alpha receptors. Such plasmapheresis can be combined with plasmapheresis of other compounds, and can enhance an immune response and an anti-inflammatory response. The reduction of circulating gal-3 will allow one of skill in the art, typically a medical practitioner with at least five (5) years of experience in the field in addition to appropriate educational experience, to more easily neutralize and inactivate the tissue expressed gal-3, thus allowing for a local immune response with less inflammation and fibrosis. As such, it can be combined with removal of TNF Alpha receptors, both R-1 and R-2. It can also be combined with administration of TNF alpha or agents that enhance TNF alpha activity.

Removing or reducing the level of circulating galectin-3 can reduce the systemic and unwanted inflammatory process, resulting, as demonstrated in the kidney MCP study, with reduction in levels of IL-6, and consequently TNF alpha and TNF Kapa Beta.

Accordingly, the method disclosed herein operates on two (2) levels:
1. Direct reduction of circulating gal-3; and
2. Ability to better target the gal-3 in the tissue level.

This has several consequences in terms of treatment effectiveness:
A. By reducing the circulating gal-3, there can be greater shedding of the tissue gal-3 through greater gradient difference, resulting in reduced inflammation, fibrosis and remodeling in the tissue level.
B. Reduction of secondary pro-inflammatory cytokines such as IL-6, TNF alpha, TNF Kapa beta, and others.
C. It can allow a greater efficacy of circulating various gal-3 blockers in general, and specifically MCP and polyuronides under 40K Dalton.
D. It can increase the efficacy of other therapies that are inhibited by excessive circulating gal-3.

In an alternative embodiment, the gal-3 comprising plasma may be run past a solid phase of immobilized gal-3 binding agents. MCP is one example and gal-3 specific antibodies, bound to a column or tube, are another. In the preferred embodiments, these two approaches to removal of gal-3 from circulation are combined. They can be combined in either order, but running the plasma past an immobile phase, followed by combining the plasma with an easily removable binding agent is preferred. Alternately the binding of an antagonist to gal-3 may be adequate to inactivate the molecule, and thus can be returned to the body without the step to remove it from the plasma.

The binding of gal-3 by a plasmapheresis element that will remove it from circulation is an event that will aid medical conditions over a wide variety of indications. On a broad scale, the indications are principally associated with inhibiting tumor growth and transformation (cancer), inflammation and fibrosis. These are implicated in specific indications such as, heart disease, kidney damage, liver damage, hepatic and renal disease, bladder disease, thyroid disease, pulmonary disease, gastrointestinal disease, immune response, stroke, persistent acute inflammation due to non-degradable pathogens, persistent foreign bodies, autoimmune reactions, hypersensitivities and allergies, pesticides, environmental toxins, and heavy metals, as well as heterogenic conditions such as radiation (examples being medical procedures such as various radiation therapies, exposure to ionizing radiation, nuclear radiation, cosmic radiation, electromagnetic radiation), chemotherapy damage, and post radiation and chemotherapy induced inflammation and fibrosis, post-surgery rise in inflammation, acute traumas such as accidents, and others.

Elevated circulating Gal-3 can change a localized situation, such as localized inflammation or fibrosis, and convert it into a larger, systemic problem. Thus, when gal-3 binds to components in the blood, which also bind toxic agents and the like, or similarly, when localized toxins are bound by gal-3, the damage potentially caused by these agents proximate to a localized injury or diseased tissue can become systemic. The same phenomenon is observed in connection with potentially metastatic cancer. Gal-3 is a generally adhesive molecule. Elevated gal-3 levels will accelerate the spread of cancer from a localized tumor to a system wide, multi-organ problem. Reducing elevated gal-3 levels below 15 or 12 ng/ml, by ten percent (10%) or more, will help to localize injury and damage, and maximize the benefit of unrelated therapeutic agents at the local injury or disease site.

As noted above, elevated gal-3 levels are associated with growth, transformation and metastatic migration of cancer cells across a wide variety of cancers, including liver cancer, kidney cancer, breast cancer, prostate cancer, colon cancer, thyroid cancer, cancer of the gallbladder, nasopharyngeal cancer, lymphocytic leukemia, lung cancer, melanoma, multiple myeloma, glioblastoma multiforme, uterine cancer, ovarian cancer, cervical cancer, and brain cancer among others, as well as reducing sensitivity in these cancers to conventional antineoplastic agents.

Elevated gal-3 levels are also associated with the development and extension of fibroses beyond normal and healthy levels, in situations associated with cardiovascular disease and heart failure, in tissue injury including brain, lungs, renal, hepatic, heart and gastroenterological situations as well as tissue damage due to radiation and chemotherapy exposure.

Above-normal gal-3 levels are also encountered in connection with inflammation. This can be disease or trauma associated inflammation, as well as persistent acute inflammation due to non-degradable pathogens, persistent foreign bodies, or autoimmune reactions, hypersensitivities and allergies, ionizing radiation, nuclear radiation and inflammation that may be associated with disease or organ failure modes, including diabetes (I and II), heart disease and dysfunction, atherosclerosis, asthma (bronchial inflammation), gastric and duodenal ulcers, intestinal inflammation in the bowels (inflammatory bowel diseases), hepatic inflammation associated with both alcohol and non-alcohol related cirrhosis and inflammation, liver infections such as viral hepatitis, among others. Other indications associated with inflammation and susceptible to treatment by plasmapheretic treatment to reduce gal-3 levels include a variety of parasite-induced conditions, such as trypanosomiasis, cerebral malaria, and inflammation and resistance to various infections including *Paracoccidiosis brasilensis* (fungal infection), schistosomiasis, granulatomatous bronchopneumonia, Lymes disease, Tubercolosis, etc. Reports of elevated gal-3 levels in connection with infection include *Candida albicans,* Reales-Calderon et al, J. Proteomics, 3:75(15) 4734-46 (2012), *Schistoma mansoni* (a parasitic infection) Brand etal, Histol. Histopathol., 27(8) 1109-20 (2012) and many others, including bacterial infections like *Neisseria meningitides,* Quattroni et al, Cell Microbiol., (Jul. 2012). Prion infection, in CNS disease, has also been linked to gal-3 elevated levels. Mok et al, Biochem. Bophys. Res. Commun. 3:359:672-8 (2007). Elevated gal-3 levels are an important contributing factor in inflammation associated with arthritis, multiple sclerosis, Parkinson's, other neurological ailments; and other diseases of the skeletomuscular and skin systems, including inflammation and fibrosis related conditions such as psoriasis and aging of the skin. See, for instance, Ezzat et al, Int. J. Rheum. Dis., 14(4):345-52 (2011) (arthritis), Gal et al, Acta. Histochem. Cytochem., 44(5):191-9 (2011) and Liu et al, Invest. Dermatol., 10.1038 (2012) (wound healing) and Larsen et al, Dermatol. Sci., 64(2):85-91 (2011) (skin diseases). As noted above, these conditions may be treated by removal of biologically active, unbound gal-3 from circulation by the use of this invention alone, or by removal from circulation combined with administration of gal-3 binding agents such as MCP to further address gal-3 mediated conditions.

Inflammation mediated at least in part by circulating gal-3 levels also plays a role in organic psychiatric and brain disorders. This kind of inflammation has been associated with a wide variety of conditions, such as schizophrenia. Muller et al., Adv. Protein Chem Struct. Biol., 88, 49-68 (2012) and Palladino et al, J. Neuroinflammation, 22;9, 206 (2012). Thus, reducing elevated gal-3 levels may be one method to assist in the control of psychiatric disorders of this type which are difficult to control by therapeutic intervention alone. Similarly, a condition receiving increasing attention, attention deficit hyperactivity disorder (ADHD) has been shown to be mediated to some degree by gal-3 expression. Wu et al, Brain Pathol., 20(6), 1042-54 (2010). Elevated gal-3 expression levels, and the inflammation associated therewith, have also been linked to organic tissue damage, as well as psychiatric behavioral disorders. Thus, Alzheimer's Disease and enhanced Aβ amyloid deposits have been shown to be associated with pro-inflammatory conditions, such as those mediated by elevated gal-3 levels. Reale, et al, Curr. Alzheimer Res. 9(4), 447-57 (2012). Gal-3 has also been shown to be involved in the proper differentiation of oligodendrocytes controlling myelin sheath conditions, Pasquin et al, Cell Death Differ., 18(11), 1746-56 (2012) and recovery and regrowth following traumatic brain injury. Venkatesan et al, J. Neuroinflammation 27(7) 32 (2010). Thus, in addition to being of importance in the control of inflammation in disease or injury conditions generally, reduction of circulating gal-3 levels through plasmapheresis may be of critical value in controlling for physical phenomena associated with disorders of the brain and central nervous system.

It should be noted that commonly, inflammation and fibrosis can be induced by deliberate treatment, not just trauma or disease condition. The removal of circulating, unbound gal-3 through the use of this invention can be effective in reducing or preventing organ damage induced by chemotherapy and other pharmaceuticals. Some examples include bleomycin, which induces lung fibroses, and a wide variety of cardiac drugs such as amiodarone. Adriamycin and doxorubicin are widely prescribed and present cardiac inflammation and fibroses issues. Bacillus Calmette-Guerin washes to treat bladder cancer induce systemic inflammation and cyclophosphamide also induces bladder damage. Cyclosporine, a widely used immunosuppressant drug, and the active agent in Restasis™, induces kidney toxicity and inflammation. Studies indicate that the vast array of organ damage caused by prescribed pharmaceuticals is mediated, at least in part, by elevated gal-3 levels, and can be limited if not eliminated by the use of this invention.

In a preferred embodiment, the serum, after having circulating gal-3 reduced or removed, as described, is further treated before returning it to the patient's blood stream. Specifically, agents that may be more effective in the absence of, or in the presence of reduced levels of, galectin-3 are specifically added. This includes a wide variety of active agents, but specifically includes agents such as chemotherapeutic drugs and therapeutic agents for the various conditions. For example, an anti-inflammatory will work better, cardiac medications, any drugs delivered to address an issue where gal-3 is a contributing factor, or prevents effective delivery to the target tissue, will be enhanced by this process. These agents will then have the opportunity to work under an environment of lower levels of gal-3. Even if just for a few hours, they can exhibit full biological activity. Once inflammation, for example, is reduced, naturally less gal-3 is being produced and expressed by the target tissue resulting in lower circulating gal-3 on a long term basis.

Thus, while in one alternative, the use of the invention involves long term or chronic plasmapheresis to maintain reduced gal-3 levels, the invention also contemplates intervention on a short term basis, both removing circulating gal-3 and providing agents otherwise inhibited by gal-3, to swiftly address inflammation in particular. Gal-3 levels can spike as a transient event, in response to trauma for example. Having a technique to rapidly lower gal-3 levels in the patient, coupled with administration of active agents that are ordinarily inhibited to some degree by high levels of gal-3, can offer a lifesaving technique.

Although Applicant does not wish to be confined to these few examples, a large number of conditions have been shown to be mediated by unbound gal-3, such that its removal, by the method disclosed herein, will aid in treatment. It has been demonstrated that reducing free gal-3 in humans can prevent renal fibrosis and inflammation following kidney injury. Both thyroid cancer and lung cancer treatment has been demonstrated to effectively improve by reducing gal-3 concentrations. Enhanced sensitivity to both radiation and chemotherapeutic intervention may be achieved by reducing circulating levels of active gal-3 through this invention.

Asthma, and related conditions primarily marked by exaggerated inflammation may be avoided or suppressed by removing circulating gal-3 through the use of this invention. These include inflammation of the gastrointestinal tract, and inflammation and the development of fibroses of the liver, interstitial cystitis, inflammation associated with brain and cognitive function, and others. Inflammation associated with parasite invasion may also be controlled by removal of gal-3, or reducing its circulating level through the use of this invention. Other inflammation-associated diseases, such as diabetes and arthritis are similarly treated. These conditions may ideally be targets of this invention as well as administration of circulating gal-3 binding agents like MCP, and unrelated therapeutic agents.

## Claims

1. A galectin-3 (gal-3) binding molecule for use in a method of treating (a) cancer; or (b) fibroses; or (c) harmful inflammation in a mammal wherein the mammal is respectively (a) in need of inhibition of the growth or spread of cancer mediated at least in part by gal-3; or (b) in need of inhibition of development or extension of fibroses mediated by gal-3; or (c) in need of inhibition of inflammation mediated by gal-3; said method comprising:
conducting plasmapheresis on the blood of a mammal in need of reduction of circulating levels of gal-3 to reduce circulating levels of active gal-3, wherein said plasmapheresis is conducted so as to selectively remove gal-3 by contact with said gal-3 binding molecule, such that at least ten percent of the circulating gal-3 in the serum of that individual is removed by said plasmapheresis; wherein the gal-3 binding molecule comprises a gal-3 binding antibody or modified citrus pectin (MCP).

2. A gal-3 binding molecule for use according to Claim 1, wherein said method further comprises periodically monitoring the level of circulating gal-3 in said mammal, and repeating said plasmapheresis on said mammal when said level of gal-3 is above a preselected level of gal-3 for said individual consistent with that individual's gal-3 mediated disease state.

3. A gal-3 binding molecule for use according to Claim 1, wherein the circulating level of gal-3 in said mammal is reduced by at least twenty-five percent (25%).

4. A gal-3 binding molecule for use according to Claim 1, wherein the circulating level of gal-3 is reduced by said plasmapheresis by about fifty percent (50%) of the level of gal-3 prior to said plasmapheresis.

5. A gal-3 binding molecule for use according to Claim 1, wherein said individual's circulating gal-3 level is reduced by said method by at least seventy-five percent (75%).

6. A gal-3 binding molecule for use according to Claim 1, wherein said step of conducting plasmapheresis comprises diverting a portion of said mammal's blood from said mammal's body to provide a volume of separated blood, removing red blood cells from said separated blood to provide separated plasma and returning said red blood cells to said mammal's circulation, contacting said separated plasma with the gal-3 binding molecule, and separating out any such gal-3 binding molecule and gal-3 bound by it from the rest of said separated plasma to provide a plasma with reduced levels of gal-3, and returning said separated plasma with reduced levels of gal-3 to said mammal's circulation.

7. A gal-3 binding molecule for use according to Claim 6 wherein said gal-3 binding molecule comprises an antibody which binds gal-3, or wherein said gal-3 binding molecule is conjugated to an element that makes separation of said gal-3 binding molecule and any gal-3 bound thereby from said separated plasma easier, wherein said element is a column to which said gal-3 binding molecule is affixed, a scaffold on which an array of said gal-3 binding molecules is affixed, magnetically attractable particles, or a mixture thereof.

8. A gal-3 binding molecule for use according to Claim 3, wherein, upon return of said separated plasma to said mammal, the level of circulating gal-3 in said mammal is below 15 ng/ml.

9. A gal-3 binding molecule for use according to Claim 1 wherein said cancer is liver cancer, kidney cancer, breast cancer, prostate cancer, colon cancer, thyroid cancer, cancer of the gallbladder, nasopharyngeal cancer, lymphocytic leukemia, lung cancer, melanoma, multiple myeloma, glioblastoma multiforme, uterine cancer, ovarian cancer, cervical cancer, or brain cancer.

10. A gal-3 binding molecule for use according to claim 1 wherein said mammal is receiving antineoplastic chemotherapy for cancer which therapy is inhibited by gal-3.

11. A gal-3 binding molecule for use according to Claim 1, wherein said fibroses are associated with cardiovascular disease, gastroenterological disease, cardiovascular trauma, brain trauma, lung trauma, renal tissue trauma, hepatic tissue trauma, tissue damage due to radiation therapy or tissue damage due to chemotherapy.

12. A gal-3 binding molecule for use according to Claim 1, wherein said inflammation:
(a) is due to pharmaceutical therapy, wherein said pharmaceutical preferably comprises bleomycin, amidoarone, Adriamycin, doxorubicin, cyclophosphamide and cyclosporine; or
(b) is: associated with non-degradable pathogens, autoimmune reactions, allergies, ionizing radiation, nuclear irradiation, diabetes, heart disease or dysfunction, atherosclerosis; bronchial inflammation; intestinal ulcers; intestinal inflammation of the bowels; hepatic inflammation; cirrhosis-associated hepatic inflammation; inflammation associated with parasitic infection; inflammation associated with viral infection; inflammation associated with fungal infection; inflammation associated with bacterial infection; arthritis associated inflammation; inflammation associated with organic psychiatric or brain disorders, multiple sclerosis, or psoriasis.

13. A gal-3 binding molecule for use according to Claim 1, wherein said mammal is in need of reduction of gal-3 levels so as to improve the effectiveness of a pharmaceutical to be administered to said individual, wherein said pharmaceutical is preferably a statin, an antineoplastic chemical agent, an anti-inflammatory agent, a TNF blocker, or a TNFα activity promoter.

14. A gal-3 binding molecule for use according to Claim 1, wherein before said serum returned to said individual it is admixed with an antineoplastic agent or an anti-inflammatory agent.

15. A gal-3 binding molecule for use according to any one of the preceding claims, wherein the gal-3 binding molecule is modified citrus pectin (MCP).

16. A gal-3 binding molecule for use according to claim 1, wherein said plasmapheresis is conducted so as to selectively remove soluble tumor necrosis factor (TNF) receptor in combination with selective removal of said gal-3 by said plasmapheresis.

17. The gal-3 binding molecule for use according to claim 16, wherein said TNF receptor is R-1 and/or R-2 and said plasmapheresis comprises running plasma fluid over a bed of binding agent of said TNF receptor.

## Patentansprüche

1. Galectin-3- (Gal-3) bindendes Molekül zur Verwendung in einem Verfahren zur Behandlung von (a) Krebs oder (b) Fibrosen oder (c) schädlichen Entzündungen in einem Säuger, wobei der Säuger jeweils (a) eine Hemmung des Wachstums oder der Ausbreitung von Krebs benötigt, der mindestens teilweise durch Gal-3 vermittelt wird, oder (b) eine Hemmung der Entwicklung oder Verlängerung von durch Gal-3 vermittelten Fibrosen benötigt oder (c) eine Hemmung der durch Gal-3 vermittelten Entzündung benötigt; wobei das Verfahren Folgendes umfasst:
Durchführen einer Plasmapherese an dem Blut eines Säugers, der eine Verringerung der zirkulierenden Spiegel von Gal-3 benötigt, um die zirkulierenden Spiegel von aktivem Gal-3 zu verringern, wobei die Plasmapherese durchgeführt wird, um Gal-3 durch Kontakt mit dem Gal-3-bindenden Molekül selektiv zu entfernen, so dass mindestens zehn Prozent des zirkulierenden Gal-3 in dem Serum dieses Individuums durch die Plasmapherese entfernt werden; wobei das Gal-3-bindende Molekül einen Gal-3-bindenden Antikörper oder modifiziertes Zitruspektin (MCP) umfasst.

2. Gal-3-bindendes Molekül zur Verwendung nach Anspruch 1, wobei das Verfahren ferner Folgendes umfasst: periodische Überwachung des Spiegels von zirkulierendem Gal-3 in dem Säuger und Wiederholung der Plasmapherese bei dem Säuger, wenn das Niveau von Gal-3 über einem vorgewählten Niveau von Gal-3 für das Individuum liegt, das mit dem Gal-3-vermittelten Krankheitszustand dieses Individuums übereinstimmt.

3. Gal-3-bindendes Molekül zur Verwendung nach Anspruch 1, wobei der zirkulierende Spiegel von Gal-3 in dem Säuger um mindestens fünfundzwanzig Prozent (25 %) verringert wird.

4. Gal-3-bindendes Molekül zur Verwendung nach Anspruch 1, wobei der zirkulierende Spiegel von Gal-3 durch die Plasmapherese um etwa fünfzig Prozent (50 %) des Spiegels von Gal-3 vor der Plasmapherese verringert wird.

5. Gal-3-bindendes Molekül zur Verwendung nach Anspruch 1, wobei die zirkulierende Gal-3-Konzentration des Individuums durch das Verfahren um mindestens fünfundsiebzig Prozent (75 %) verringert wird.

6. Gal-3-bindendes Molekül zur Verwendung nach Anspruch 1, wobei der Schritt der Durchführung der Plasmapherese Folgendes umfasst: Umleiten eines Teils des Blutes des Säugers aus dem Körper des Säugers, um ein Volumen von getrenntem Blut bereitzustellen, Entfernen von roten Blutkörperchen aus dem getrennten Blut, um getrenntes Plasma bereitzustellen, und Zurückführen der roten Blutzellen in den Kreislauf des Säugers, Kontaktieren des getrennten Plasmas mit dem Gal-3-bindenden Molekül und Trennen von allen Gal-3-bindenden Molekülen und allem daran gebundenem Gal-3 von dem Rest des abgetrennten Plasmas, um ein Plasma mit verringerten Mengen an Gal-3 bereitzustellen, und Zurückführen des abgetrennten Plasmas mit verringerten Mengen an Gal-3 in den Kreislauf des Säugers.

7. Gal-3-bindendes Molekül zur Verwendung nach Anspruch 6, wobei das Gal-3-bindende Molekül einen Antikörper umfasst, der Gal-3 bindet, oder wobei das Gal-3-bindende Molekül an ein Element konjugiert ist, das eine Trennung des Gal-3-bindenden Moleküls und etwaigem gebundenem Gal-3, das dadurch gebunden ist, aus dem abgeschiedenen Plasma erleichtert, wobei das Element Folgendes ist: eine Säule, an die das Gal-3-bindende Molekül gebunden ist, ein Gerüst, auf dem eine Anordnung der Gal-3-bindenden Moleküle angebracht ist, magnetisch anziehbare Teilchen oder eine Mischung davon.

8. Gal-3-bindendes Molekül zur Verwendung nach Anspruch 3, wobei bei der Rückführung des abgetrennten Plasmas in den Säuger der Gehalt an zirkulierendem Gal-3 im Säuger unter 15 ng/ml liegt.

9. Gal-3-bindendes Molekül zur Verwendung nach Anspruch 1, wobei der Krebs Folgendes ist: Leberkrebs, Nierenkrebs, Brustkrebs, Prostatakrebs, Darmkrebs, Schilddrüsenkrebs, Gallenblasenkrebs, Nasopharynxkrebs, Lymphozytenleukämie, Lungenkrebs, Melanom, multiples Myelom, Glioblastoma multiforme, Gebärmutterkrebs, Eierstockkrebs, Gebärmutterhalskrebs, oder Hirntumor.

10. Gal-3-bindendes Molekül zur Verwendung nach Anspruch 1, wobei der Säuger antineoplastische Chemotherapie für Krebs erhält, wobei die Therapie durch Gal-3 inhibiert wird.

11. Gal-3-bindendes Molekül zur Verwendung nach Anspruch 1, wobei die Fibrosen Folgendem zugeordnet sind: kardiovaskulärer Erkrankung, gastroenterologischer Erkrankung, kardiovaskulärem Trauma, Hirntrauma, Lungentrauma, Nierentrauma, Lebertrauma, Gewebeschädigung durch Strahlentherapie oder Gewebeschädigung durch Chemotherapie.

12. Gal-3-bindendes Molekül zur Verwendung nach Anspruch 1, wobei die Entzündung:
(a) auf eine pharmazeutische Therapie zurückzuführen ist, wobei das Pharmazeutikum vorzugsweise Bleomycin, Amidoaron, Adriamycin, Doxorubicin, Cyclophosphamid und Cyclosporin umfasst; oder
(b) im Zusammenhang mit Folgendem steht: nicht abbaubaren Krankheitserregern, Autoimmunreaktionen, Allergien, ionisierender Strahlung, nuklearer Bestrahlung, Diabetes, Herzerkrankungen oder Dysfunktion, Atherosklerose; bronchialer Entzündung; intestinalen Geschwüren; Darmentzündung des Darms; Leberentzündung; Leberzirrhosezugeordneter Leberentzündung; Entzündung im Zusammenhang mit parasitären Infektionen; Entzündung im Zusammenhang mit viraler Infektion; Entzündung im Zusammenhang mit Pilzinfektion; Entzündung im Zusammenhang mit einer bakteriellen Infektion; Arthritis-zugeordneter Entzündung; Entzündung im Zusammenhang mit organischen psychiatrischen oder Hirnerkrankungen, Multipler Sklerose oder Psoriasis.

13. Gal-3-bindendes Molekül zur Verwendung nach Anspruch 1, wobei der Säuger eine Verringerung der Gal-3-Spiegel benötigt, um die Wirksamkeit eines dem Individuum zu verabreichenden Arzneimittels zu verbessern, wobei das Pharmazeutikum vorzugsweise ein Statin, ein antineoplastisches chemisches Mittel, ein entzündungshemmendes Mittel, ein TNF-Blocker oder ein TNFa-Aktivitätspromotor ist.

14. Gal-3-bindendes Molekül zur Verwendung nach Anspruch 1, wobei das Serum, bevor es zu dem Individuum zurückkehrt, mit einem antineoplastischen Mittel oder einem entzündungshemmenden Mittel vermischt wird.

15. Gal-3-bindendes Molekül zur Verwendung nach einem der vorhergehenden Ansprüche, wobei das Gal-3-bindende Molekül modifiziertes Citruspektin (MCP) ist.

16. Gal-3-bindendes Molekül zur Verwendung nach Anspruch 1, wobei die Plasmapherese durchgeführt wird, um den löslichen Tumor-Nekrose-Faktor- (TNF) Rezeptor in Kombination mit einer selektiven Entfernung von Gal-3 durch die Plasmapherese selektiv zu entfernen.

17. Gal-3-bindendes Molekül zur Verwendung nach Anspruch 16, wobei der TNF-Rezeptor R-1 und/oder R-2 ist und die Plasmapherese das Fließen von Plasmaflüssigkeit über ein Bett eines Bindemittels des TNF-Rezeptors umfasst.

## Revendications

1. Molécule de liaison à la galectine 3 (gal-3) pour l'utilisation dans un procédé de traitement (a) du cancer ; ou (b) de fibroses ; ou (c) d'inflammation nocive chez un mammifère, dans laquelle le mammifère nécessite respectivement (a) l'inhibition de la croissance ou de la propagation de cancer à médiation au moins partielle par gal-3 ; ou (b) l'inhibition de développement ou d'extension de fibroses à médiation par gal-3 ; ou (c) l'inhibition d'inflammation à médiation par gal-3 ; ledit procédé comprenant :
la réalisation de plasmaphérèse sur le sang d'un mammifère nécessitant une réduction de niveaux circulants de gal-3 pour réduire des niveaux circulants de gal-3 active, dans laquelle ladite plasmaphérèse est réalisée afin de sélectivement éliminer la gal-3 par contact avec ladite molécule de liaison à la gal-3, de telle sorte qu'au moins dix pour cent de la gal-3 circulante dans le sérum de cet individu soit éliminée par ladite plasmaphérèse ; dans laquelle la molécule de liaison à la gal-3 comprend un anticorps ou une pectine d'agrume modifiée (MCP) de liaison à la gal-3.

2. Molécule de liaison à la gal-3 pour l'utilisation selon la revendication 1, dans laquelle ledit procédé comprend en outre la surveillance périodique du niveau de gal-3 circulante chez ledit mammifère, et la répétition de ladite plasmaphérèse sur ledit mammifère lorsque ledit niveau de gal-3 est supérieur à un niveau présélectionné de gal-3, pour ledit individu, conforme à l'état de maladie à médiation par gal-3 de cet individu.

3. Molécule de liaison à la gal-3 pour l'utilisation selon la revendication 1, dans laquelle le niveau circulant de gal-3 chez ledit mammifère est réduit d'au moins vingt cinq pour cent (25 %).

4. Molécule de liaison à la gal-3 pour l'utilisation selon la revendication 1, dans laquelle le niveau circulant de gal-3 est réduit par ladite plasmaphérèse d'environ cinquante pour cent (50 %) du niveau de gal-3 avant ladite plasmaphérèse.

5. Molécule de liaison à la gal-3 pour l'utilisation selon la revendication 1, dans laquelle le niveau de gal-3 circulante dudit individu est réduit, par ledit procédé, d'au moins soixante-quinze pour cent (75 %).

6. Molécule de liaison à la gal-3 pour l'utilisation selon la revendication 1, dans laquelle ladite étape de la réalisation de plasmaphérèse comprend le détournement d'une portion du sang dudit mammifère à partir du corps dudit mammifère pour fournir un volume de sang séparé, l'élimination de globule rouges à partir dudit sang séparé pour fournir un plasma séparé et la réintroduction desdits globules rouges dans la circulation dudit mammifère, la mise en contact dudit plasma séparé avec la molécule de liaison à la gal-3, et la séparation d'une quelconque telle molécule de liaison à la gal-3 et de la gal-3 à laquelle elle s'est liée du reste dudit plasma séparé pour fournir un plasma avec des niveaux réduits de gal-3, et la réintroduction dudit plasma séparé, avec des niveaux réduits de gal-3, dans la circulation dudit mammifère.

7. Molécule de liaison à la gal-3 pour l'utilisation selon la revendication 6, dans laquelle ladite molécule de liaison à la gal-3 comprend un anticorps qui se lie à la gal-3, ou dans laquelle ladite molécule de liaison à la gal-3 est conjuguée à un élément qui facilite la séparation de ladite molécule de liaison à la gal-3 et d'une quelconque gal-3 à laquelle elle est liée dudit plasma séparé, dans laquelle ledit élément est une colonne, à laquelle ladite molécule de liaison à la gal-3 est fixée, un échafaudage, sur lequel une série desdites molécules de liaison à la gal-3 est fixée, des particules pouvant faire l'objet d'une attraction magnétique, ou un mélange de ceux-ci.

8. Molécule de liaison à la gal-3 pour l'utilisation selon la revendication 3, dans laquelle, lors de la réintroduction dudit plasma séparé dans ledit mammifère, le niveau de gal-3 circulante chez ledit mammifère est inférieur à 15 ng/ml.

9. Molécule de liaison à la gal-3 pour l'utilisation selon la revendication 1, dans laquelle ledit cancer est le cancer du foie, le cancer du rein, le cancer du sein, le cancer de la prostate, le cancer du colon, le cancer de la thyroïde, le cancer de la vésicule biliaire, le cancer nasopharyngien, la leucémie lymphatique, le cancer du poumon, le mélanome, le myélome multiple, le glioblastome multiforme, le cancer de l'utérus, le cancer de l'ovaire, le cancer du col de l'utérus, ou le cancer du cerveau.

10. Molécule de liaison à la gal-3 pour l'utilisation selon la revendication 1, dans laquelle ledit mammifère reçoit une chimiothérapie antinéoplasique pour le cancer, laquelle thérapie est inhibée par la gal-3.

11. Molécule de liaison à la gal-3 pour l'utilisation selon la revendication 1, dans laquelle lesdites fibroses sont associées à une maladie cardiovasculaire, une maladie gastroentérologique, un trauma cardiovasculaire, un trauma cérébral, un trauma pulmonaire, un trauma du tissu rénal, un trauma du tissu hépatique, une lésion tissulaire due à la radiothérapie ou une lésion tissulaire due à la chimiothérapie.

12. Molécule de liaison à la gal-3 pour l'utilisation selon la revendication 1, dans laquelle ladite inflammation :
(a) est due à une thérapie pharmaceutique, dans laquelle ledit produit pharmaceutique comprend de préférence la bléomycine, l'amidoarone, l'adriamycine, la doxorubicine, la cyclophosphamide et la cyclosporine ; ou
(b) est : associée à des pathogènes non dégradables, des réactions auto-immunes, des allergies, un rayonnement ionisant, une irradiation nucléaire, un diabète, une maladie ou un trouble cardiaque, l'athérosclérose ; une inflammation bronchiale ; des ulcères intestinaux ; une inflammation intestinale ; une inflammation hépatique ; une inflammation hépatique associée à la cirrhose ; une inflammation associée à une infection parasitique ; une inflammation associée à une infection virale ; une inflammation associée à une infection fongique ; une inflammation associée à une infection bactérienne ; une inflammation associée à l'arthrite ; une inflammation associée à des troubles psychiatriques ou cérébraux organiques, la sclérose en plaques, ou le psoriasis.

13. Molécule de liaison à la gal-3 pour l'utilisation selon la revendication 1, dans laquelle ledit mammifère nécessite une réduction de niveau de gal-3 afin d'améliorer l'efficacité d'un produit pharmaceutique destiné à être administré audit individu, dans laquelle ledit produit pharmaceutique est de préférence une statine, un agent chimique antinéoplasique, un agent anti-inflammatoire, un anti-TNF, ou un promoteur d'activité TNFα.

14. Molécule de liaison à la gal-3 pour l'utilisation selon la revendication 1, dans laquelle avant que ledit sérum soit réintroduit dans ledit individu il est mélangé avec un agent antinéoplasique ou un agent anti-inflammatoire.

15. Molécule de liaison à la gal-3 pour l'utilisation selon l'une quelconque des revendications précédentes, dans laquelle la molécule de liaison à la gal-3 est de la pectine d'agrume modifiée (MCP).

16. Molécule de liaison à la gal-3 pour l'utilisation selon la revendication 1, dans laquelle ladite plasmaphérèse est réalisée afin de sélectivement éliminer un récepteur de facteur de nécrose tumorale (TNF) soluble en association avec l'élimination sélective de ladite gal-3 par ladite plasmaphérèse.

17. Molécule de liaison à la gal-3 pour l'utilisation selon la revendication 16, dans laquelle ledit récepteur de TNF est R-1 et/ou R-2 et ladite plasmaphérèse comprend l'écoulement d'un fluide plasmatique sur un lit d'agent liant dudit récepteur de TNF.
